## Europäisches Patentamt
## European Patent Office
## Office européen des brevets

(19)

(11) Numéro de publication: **0 049 673**
**B1**

(12)

# FASCICULE DE BREVET EUROPEEN

(45) Date de publication du fascicule du brevet:
25.09.85

(51) Int. Cl.⁴: **A 61 M 1/14**

(21) Numéro de dépôt: **81420138.0**

(22) Date de dépôt: **23.09.81**

(54) Rein artificiel avec circuit de liquide de dialyse à usage unique.

(30) Priorité: **06.10.80 FR 8021781**

(43) Date de publication de la demande:
**14.04.82 Bulletin 82/15**

(45) Mention de la délivrance du brevet:
**25.09.85 Bulletin 85/39**

(84) Etats contractants désignés:
**BE CH DE GB IT LI NL SE**

(56) Documents cités:
**FR - A - 1 566 244**
**FR - A - 2 082 978**
**FR - A - 2 085 298**
**FR - A - 2 093 706**
**FR - A - 2 242 994**
**FR - A - 2 424 743**
**US - A - 3 515 275**

(73) Titulaire: **HOSPAL INDUSTRIE, 7, Avenue Lionel Terray, F-69330 Meyzieu (FR)**

(72) Inventeur: **Vantard, Georges, 20, rue des Prés de Noisy, F-93460-Gournay sur Marne (FR)**

(74) Mandataire: **Gauckler, Jacques et al, RHONE-POULENC RECHERCHES HOSPAL-BREVETS Centre de Recherches de Saint-Fons B.P. 62, F-69190 Saint-Fons (FR)**

ACTORUM AG

## Description

La présente invention concerne un rein artificiel. Elle concerne plus particulièrement un rein artificiel d'un type amélioré et simplifié, dans lequel les éléments constituant le circuit hydraulique parcouru par le liquide de dialyse sont conçus, fabriqués et assemblés en vue d'un usage unique.

Un rein artificiel comprend en général:

a) un hémodialyseur divisé par une membrane permettant le traitement du sang par dialyse et par ultrafiltration en deux compartiments, le premier parcouru par le sang, le second par le liquide de dialyse,

b) des moyens pour faire circuler le sang traité dans le premier compartiment,

c) des moyens pour préparer le liquide de dialyse, le stocker et le faire circuler dans le second compartiment,

d) des moyens pour prélever et mesurer des quantités de liquide égales aux quantités d'ultrafiltrat désirées,

e) des organes de commande et de contrôle desdits moyens selon b) et c).

Les moyens selon a) et b), constitués d'éléments dans lesquels circulent le sang, ne sont en général pas réutilisés pour des raisons évidentes de sécurité. Ils sont donc conçus pour un usage unique et l'on a déjà proposé de les associer dans un ensemble à usage unique, afin d'en réduire le coût et d'en faciliter l'utilisation.

Au contraire, les moyens selon c) et d) ne sont généralement réutilisables qu'après stérilisation. Aussi font-ils le plus souvent partie d'un appareillage appelé moniteur, conçu pour des usages nombreux et répétés, regroupant également les divers organes selon e) reliés électriquement pour la commande, le contrôle et la sécurité du traitement. Il est bien connu que la fabrication des moyens selon c) et d) est fort coûteuse et que leur mise en œuvre nécessite un personnel qualifié.

Certes on a bien cherché à supprimer tout traitement préalable de stérilisation en utilisant pour le liquide de dialyse des lignes à usage unique. Mais on n'a fait que remplacer dans des appareillages connus, une tuyauterie supportant des réutilisations répétées par une tuyauterie à usage unique, tout en conservant les autres organes existants.

Du FR-A-2 085 298 est connu un ensemble d'éléments à usage unique, destiné aux hémodialyses, dans lequel le circuit complet pouvant être branché entre les arrivées des deux composants du liquide électrolytique et l'entrée du dialyseur et le dialyseur lui-même sont jetables.

Du FR-A-2 082 978 est connu un module à dialysat à remise en circulation, destiné à être jeté. Ce module comprend un récipient en matière plastique, qui contient tous les produits chimiques, les fluides, les chambres, les réservoirs, les cavités, les passages, les pompes à fluide, les jauges et soupapes et les connexions nécessaires pour mettre convenablement en circulation et traiter un fluide de dialysat entre la sortie et l'entrée d'un rein artificiel.

Un objet de la présente invention est de proposer un rein artificiel d'un type nouveau, amélioré et simplifié, en vue notamment d'un usage unique des moyens pour préparer le liquide de dialyse, le stocker et le faire circuler.

Un autre objet de la présente invention est de proposer un rein artificiel dont la plupart des éléments se prêtent à une fabrication économique en grande série.

Un autre objet de la présente invention est de réduire très sensiblement le coût de l'immobilisation du matériel d'hémodialyse réutilisable après chaque traitement.

Un autre objet de la présente invention est d'accélérer et de faciliter très sensiblement la mise en œuvre d'un rein artificiel, tout en augmentant la sécurité d'emploi, permettant ainsi un développement de la dialyse à domicile.

Un autre objet de la présente invention est de réduire sensiblement le coût global d'une séance d'hémodialyse, permettant ainsi une extension du marché de l'hémodialyse.

D'autres objets de la présente invention apparaîtront au cours de la description qui va suivre.

Il a maintenant été trouvé un rein artificiel comprenant:

a) un hémodialyseur divisé par une membrane permettant le traitement du sang par dialyse et ultrafiltration en deux compartiments, le premier parcouru par le sang, le second par le liquide de dialyse,

b) des moyens pour faire circuler le sang traité dans ledit premier compartiment,

c) des moyens pour préparer le liquide de dialyse, le stocker et le faire circuler dans ledit second compartiment,

d) des moyens pour prélever et mesurer des quantités de liquide égales aux quantités d'ultrafiltrat désirées,

e) des organes de commande et de contrôle desdits moyens selon b) et c), réutilisables et groupés sur une console (10) susceptible d'être reliée à une source de courant électrique, caractérisé en ce que lesdits moyens selon c) comprennent une cartouche à usage unique (20) de concentré de dialyse d'une capacité calibrée pour un traitement donné, apte à être raccordée à une source de distribution d'eau courante.

La compréhension de l'invention sera facilitée par les figures ci-jointes qui représentent schématiquement et sans échelle déterminée divers exemples de réalisation.

La figure 1 est une vue en perspective d'un mode de réalisation particulier de l'ensemble du rein artificiel selon l'invention.

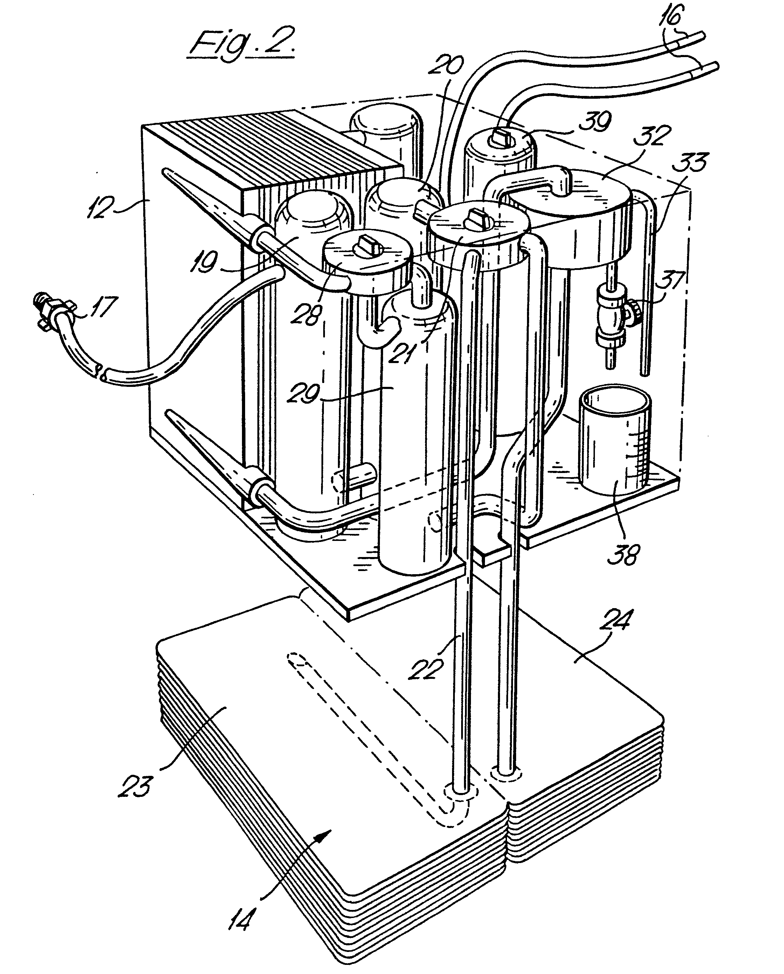

La figure 2 est une vue en perspective des éléments à usage unique raccordés à l'hémodialyseur d'un rein artificiel semblable à celui représenté figure 1.

La figure 3 est la vue de dessus des éléments de la figure 2.

La figure 4 est la vue en coupe selon III–III de la figure 3 de l'ensemble des éléments à usage unique.

La figure 5 est la vue en coupe selon III–III de la figure 3 de l'ensemble du rein artificiel selon la figure 1, prêt à fonctionner.

La figure 6 est un schéma de circulation du liquide de dialyse dans un mode de réalisation particulier du rein artificiel selon l'invention.

Selon une caractéristique de la présente invention, les moyens pour préparer le liquide de dialyse, le stocker et le faire circuler peuvent être à usage unique, grâce à d'importantes simplifications qui se traduisent le plus souvent par des suppressions d'organes.

Ainsi, contrairement aux méthodes habituelles, on a trouvé possible et avantageux de refouler le liquide de dialyse dans l'hémodialyseur à une pression légèrement supérieure à la pression atmosphérique, quoique restant constamment inférieure à la pression du sang grâce à un dispositif d'autorégulation approprié, ce qui permet de supprimer des manomètres, et d'éviter ou de réduire considérablement les problèmes de dégazage et des risques de rentrée d'air.

En outre, le liquide de dialyse et l'ultrafiltrat retiré au patient sont déplacés selon des méthodes volumétriques, ce qui permet de supprimer toute pesée de liquide, du patient ou de tout ou partie de l'appareillage. De même l'usage d'un volume prédéterminé de liquide de dialyse préalablement préparé permet de se passer d'organes de mesure permanent de la conductivité de ce liquide, sans risque pour la sécurité du patient.

De plus, suite à la suppression de toute stérilisation, disparaissent, avec les organes et les dispositions liés à son usage, les opérations de stérilisation elles-mêmes, ainsi que les risques inhérents à cette technique.

Selon une autre caractéristique de la présente invention, l'ensemble des éléments à usage unique nécessaire à une séance d'hémodialyse, comprenant notamment les moyens pour préparer le liquide de dialyse, le stocker et assurer sa circulation, présente un encombrement dont le volume correspond, de préférence, précisément au volume total de liquide de dialyse nécessaire à une séance, soit une trentaine de litres seulement environ.

Ceci présente l'avantage de permettre de disposer l'ensemble des éléments à usage unique nécessaire à une séance d'hémodialyse dans un container (11), indéformable sous l'effet des contraintes habituelles, utilisable pour de multiples fonctions ce qui, de préférence, permet de le fabriquer en vue d'un usage unique.

En effet, ce container (11) permet d'assurer la contention, le transport, le stockage et la livraison de l'ensemble des éléments à usage unique nécessaire à une séance, avant et/ou après ladite séance. Il assure de plus, pendant la séance, la délimitation et la contention sans déformation sensible du volume de liquide de dialyse frais et/ou usagé exactement nécessaire au traitement. De préférence il maintient pendant la séance le liquide de dialyse à des températures voisines de celles du sang. En outre, il peut avantageusement servir pendant la séance de support à l'ensemble du matériel constituant le rein artificiel.

Après chaque séance, l'équipement à usage unique peut alors être soit jeté directement, soit remis en place dans le container, l'ensemble ainsi constitué pouvant être à son tour, soit jeté, soit échangé contre un ensemble neuf identique, ce qui réduit considérablement les manutentions, les risques d'erreur, pertes ou détériorations. Le cas échéant le container peut être retourné en atelier, traité, vérifié et recyclé.

On a en effet tiré parti du fait que l'on peut avantageusement procéder à des traitements selon des taux d'épuration relativement modérés, définis par exemple par une clairance hebdomadaire en urée de 70 à 90 litres et en vitamine B 12 de 20 à 30 litres. De tels traitements sont possibles, notamment avec des hémodialyseurs de surface réduite à 0,5 m² équipés de membranes telles que décrites dans les brevets FR-A-2 105 502 et FR-A-2 199 014. Il en résulte qu'avec un rein artificiel selon l'invention il est possible de ramener le volume total de liquide de dialyse d'environ 200 à 300 litres à un volume inférieur à 80 litres environ.

Le volume de liquide de dialyse peut en outre être encore réduit et être ajusté à des valeurs d'environ 30 litres, soit en procédant à une épuration partielle du liquide de dialyse recyclé, à l'aide de passages sur produits adsorbants tels que le charbon actif, soit en opérant à faible débit de liquide de dialyse, sans recirculation ni mélange du liquide usagé avec le liquide frais, comme décrit ci-après.

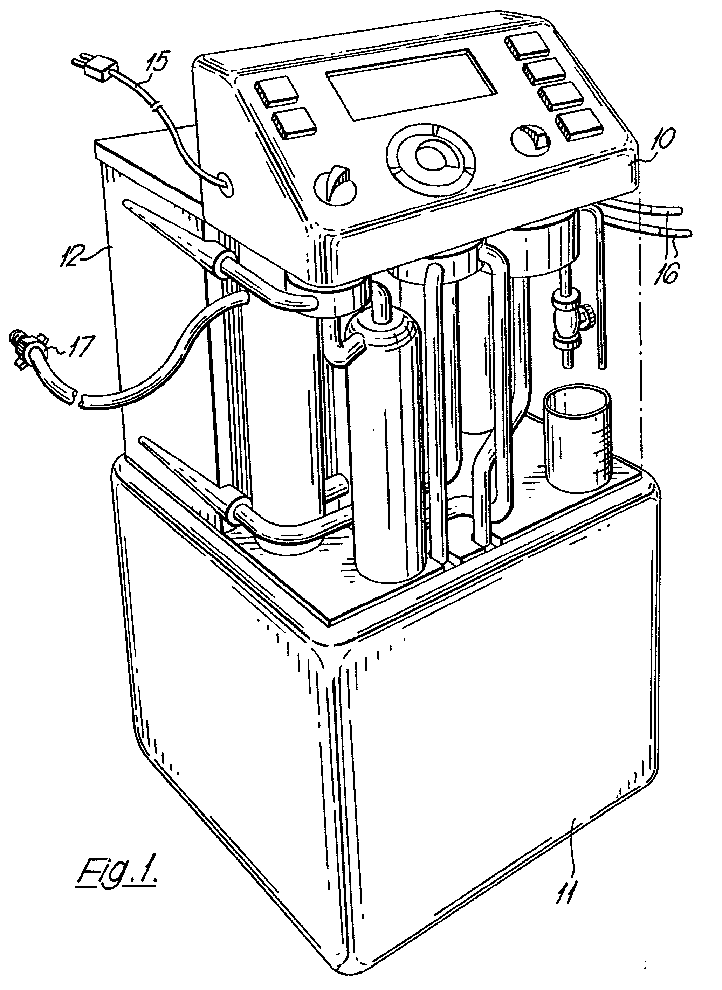

Selon une autre caractéristique de la présente invention, le rein artificiel représenté figure 1 est entièrement composé d'éléments à usage unique à l'exception d'une petite console (10) qui, disposée à la partie supérieure, ne regroupe plus que des organes de commande et de contrôle du rein artificiel, ainsi que les circuits électriques correspondants susceptibles d'être reliés à une source de courant électrique extérieure au moyen d'une ligne (15).

On remarque, et ceci est une caractéristique importante de la présente invention, que cette console est dépourvue de tout circuit hydraulique, notamment pour la préparation ou la circulation du liquide de dialyse, ainsi que pour le prélèvement et le contrôle de quantités de liquide égales aux quantités d'ultrafiltrat désirées. Le fait que les circuits électriques de la console ne soient plus mêlés à des circuits hydrauliques constitue une amélioration très importante pour la sécurité de fonctionnement du rein artificiel.

Il ressort également de ce fait que l'encombrement de la console, pratiquement seul élément du rein artificiel réutilisable d'un traitement à l'autre, est minime, que sa manipulation est aisée et que l'investissement initial est considérablement réduit.

Par ailleurs, mais de façon conventionnelle, les éléments à usage unique sont généralement livrés à l'utilisateur avant chaque séance, préalablement assemblés, contrôlés et stérilisés, sous emballage étanche, prêts à l'emploi.

D'autres caractéristiques et d'autres avantages de la présente invention apparaîtront au cours de la description qui va suivre des exemples de réalisation correspondant aux figures.

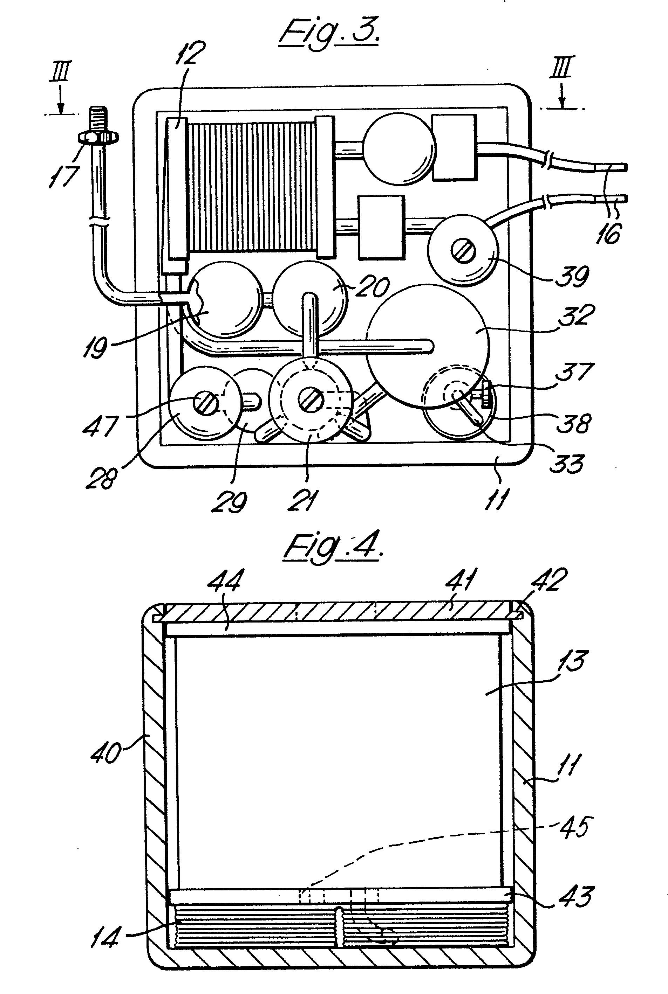

Selon la figure 3, l'ensemble de l'équipement à usage unique est disposé en quatre zones délimitées approximativement par les plans de symétrie du container (11). On a en effet en haut à gauche, puis successivement dans le sens des aiguilles d'une montre:

— l'hémodialyseur (12),

— les moyens constituant le circuit sang reliant l'hémodialyseur au patient, via la pompe à sang (39) et les canules (16),

— les moyens (37, 38) pour prélever et contrôler des quantités de liquide de dialyse égales aux quantités d'ultrafiltrat désirées,

— enfin les moyens pour la préparation et la circulation du liquide de dialyse.

Tous ces moyens apparaissent figure 1, entre la console (10) et le container (11). Figure (2) est représentée en outre une poche souple (14) étanche, vide et pliée sur elle-même, destinée à contenir le volume de liquide de dialyse exactement nécessaire.

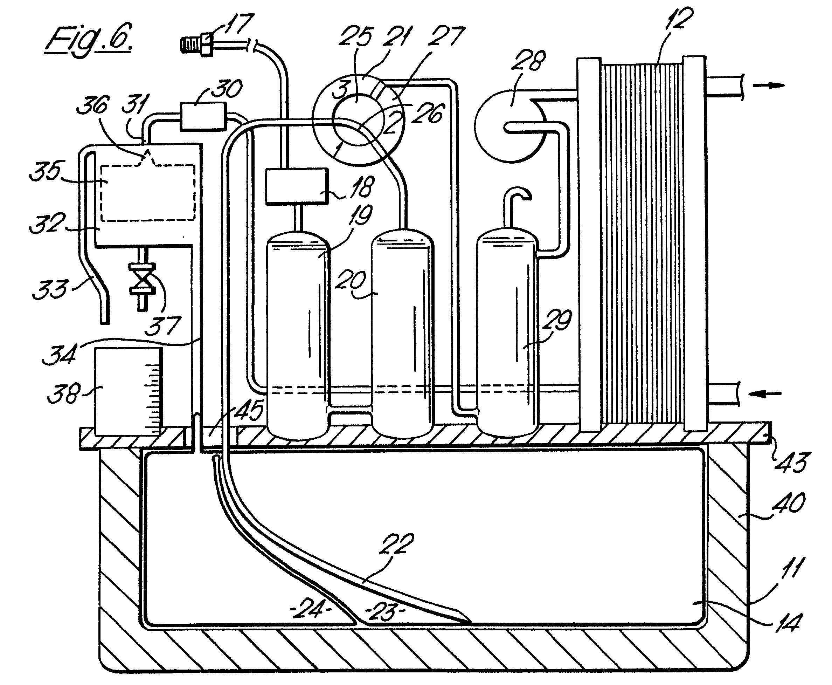

La figure 4 représente un mode de réalisation particulier d'un container (11) comprenant une cuve (40) et un couvercle (41) coulissant dans deux glissières parallèles telles que (42). A l'intérieur prend place l'équipement à usage unique schématisé par le rectangle (13) ainsi que la poche de stockage (14). Deux plaques parallèles (43) et (44) servent à maintenir assemblés en position les divers éléments à usage unique. Ces plaques, à usage unique, peuvent être solidarisées par tous moyens, par exemple par une tige de liaison (non représentée) vissée en leurs milieux respectifs. La plaque inférieure (43) est munie d'une ouverture (45) permettant le passage des conduits (22) et (34). Les divers éléments à usage unique peuvent être solidarisés de ces plaques, comme indiqué figure 6 pour la plaque inférieure (43).

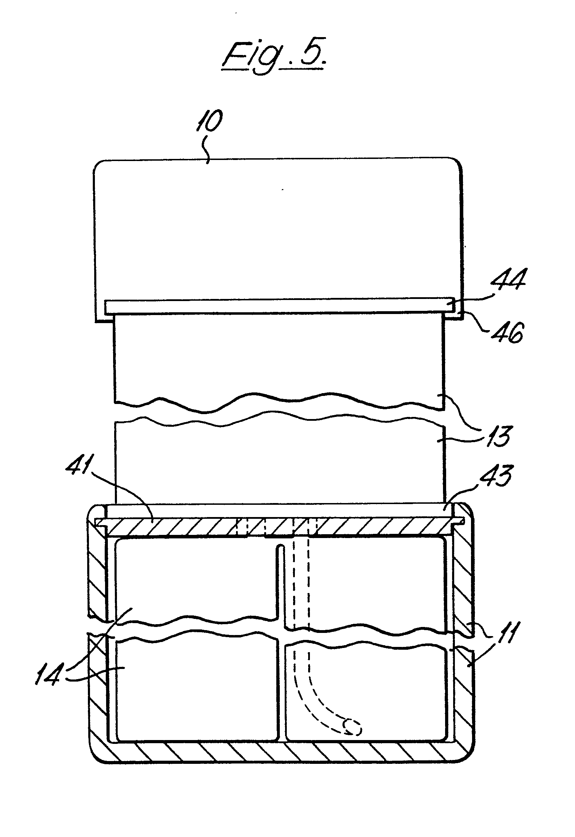

Figure 5, le couvercle (41) a été retiré pour permettre de sortir hors du container l'équipement à usage unique. Le couvercle a été ensuite replacé dans ses glissières après avoir été retourné, pour permettre à cet équipement d'être posé sur le container et à la plaque inférieure (43) de pouvoir s'emboîter exactement à l'intérieur du container, sur le couvercle.

La console (10) est avantageusement munie de deux glissières latérales telles que (46) coopérant avec les bords correspondants de la plaque supérieure (44). La console (10) est donc introduite par translation sur la plaque (44) jusqu'à ce que les divers organes d'accouplement mécanique correspondant par exemple d'une part aux pompes et aux vannes à usage unique, et d'autre part à leurs moyens d'entraînement réutilisables (moteurs, volants) viennent en prise, par exemple à l'aide de carrés d'entraînement s'emboîtant dans des dispositifs femelles correspondants. Il suffit alors de procéder aux raccords nécessaires (circuits électriques) et de vérifier que toutes les liaisons sont correctement effectuées.

L'hémodialyseur est divisé par une membrane permettant la dialyse et l'ultrafiltration du sang en un premier compartiment parcouru par le sang et un second compartiment parcouru par le liquide de dialyse, de préférence à contre courant. L'hémodialyseur peut être de différents types connus et la surface d'échanges peut avoir toutes valeurs usuelles. Un hémodialyseur à fibres creuses convient bien, cependant on préfère les hémodialyseurs à membrane de forme générale plane ou pliée autour d'intercalaires plans, pleins ou ajourés, car leur géométrie assez précise est favorable aux échanges avec faible débit de liquide de dialyse (low-flow) et le dégazage peut s'effectuer sans retourner l'appareil.

Les moyens à usage unique constituant le circuit parcouru par le sang et reliant l'hémodialyseur au patient sont également de tous types connus et ne seront donc pas décrits plus en détails.

Un exemple de réalisation des moyens pour préparer, stocker et faire circuler le liquide de dialyse, ainsi que pour contrôler l'ultrafiltrat à prélever au patient est représenté schématiquement figure 6.

Ce circuit à usage unique peut être raccordé par un embout (17) à tout circuit de distribution d'eau courante. Cette eau peut être fournie, soit à température ambiante, soit de préférence à une température comprise approximativement entre 35°C et 40°C par mélange préalable eau froide-eau chaude, contrôlé par thermostat.

Le circuit à usage unique comporte avantageusement un regard (18) transparent permettant de vérifier l'écoulement de l'eau introduite. Le cas échéant ce regard peut être muni d'un clapet taré qui limite automatiquement la pression de l'eau dans le circuit à usage unique.

Ce circuit peut comporter également un dispositif adoucisseur (19) à usage unique muni par exemple de résines échangeuses d'ions de types connus en soi ou de membranes d'osmose inverse sur cordonnets selon le brevet FR-A-2 276 855.

Il comporte ensuite un réservoir (20) à usage unique contenant une cartouche de concentré de dialyse, sous forme pulvérulente ou de préférence sous forme de solution concentrée, dont la capacité (en poids ou volume) est préalablement calibrée pour correspondre exactement à la capacité du circuit. Une solution concentrée d'environ un litre est nécessaire pour préparer 30 litres de liquide de dialyse, elle doit être définie avec une précision de +25 ml.

Ce concentré peut être par exemple contenu dans une poche plastique scellée de manière étanche et munie d'un clapet taré solidaire d'un poinçon axial susceptible de perforer la paroi opposée de la poche. Sous l'effet de la pression de l'eau, le clapet se déplace et le poinçon perfore la poche, ce qui permet simultanément l'introduction de l'eau dans la poche et l'évacuation du mélange d'eau et de concentré en aval de la poche.

Ce mélange est alors envoyé, via un robinet 3 voies (21) et un tube plongeur (22) au fond du container (11) jusqu'à le remplir entièrement.

On a trouvé que lorsqu'on envoie le mélange eau-concentré de dialyse au fond du container, le mélange devient rapidement homogène sous l'effet des courants de convexion, sans intervention mécanique. Il devient aussi superflu de prévoir un conductivimètre pour mesurer, contrôler ou règler la conductivité du mélange, ce qui constitue au total une sensible économie.

Selon un mode de réalisation particulier, le container, muni de parois indéformables sous les contraintes habituelles, peut contenir, comme représenté, une poche (14) souple et étanche aux fluides, à usage unique. Cette poche souple peut être divisée en deux compartiments complémentaires (23) et (24), susceptibles d'occuper chacun tout le volume interne disponible. L'un de ces compartiments (23) peut recevoir et contenir le liquide de dialyse frais, et l'autre (24) le liquide de dialyse usagé.

Le robinet trois voies (21) est avantageusement constitué par un cylindre (25) muni d'un canal (26) susceptible de tourner de manière étanche à l'intérieur d'un corps (27) muni de trois orifices radiaux orientés de préférence à 120°, pour pouvoir mettre en communication deux quelconques de ces trois orifices. Ce robinet est représenté en position 1, c'est-à-dire reliant les deux orifices disposés de part et d'autre du repère 1.

Ainsi, lorsque le compartiment (23) est plein, c'est-à-dire lorsqu'on constate l'arrêt de l'écoulement de l'eau dans le regard (18), on tourne le robinet (21) d'un tiers de tour pour le placer de la position 1 à la position 2. On effectue le remplissage initial du circuit de liquide de dialyse comprenant l'hémodialyseur, à l'aval du robinet trois voies, avec de l'eau, sans pratiquement consommer de concentré de dialyse. La commande du robinet s'effectue généralement à partir de la console (10) grâce à un dispositif d'entraînement s'accouplant mécaniquement au cylindre (25) lors de la mise en place de la console.

L'eau s'écoule alors à travers la pompe (28) et l'hémodialyseur (12). Le cas échéant l'eau traverse un dispositif conventionnel de chauffage (29) par circulation d'eau chaude à partir du robinet de distribution d'eau courante ou par infrarouges, complété par un dispositif de régulation de température, les dispositifs électriques étant solidaires de la console (10) et n'étant pas en contact avec les liquides. Le dispositif (29) peut éventuellement comporter un dispositif de dégazage.

La pompe (28) est de type conventionnel, par exemple péristaltique, centrifuge, à engrenages, etc... Elle est entraînée en rotation par un axe (47) susceptible de s'accoupler à l'axe d'un moteur électrique d'entraînement logé dans la console (10). Le plus souvent, un moteur similaire, également logé dans la console (10), entraîne par un accouplement approprié la pompe (39) qui déplace le sang du patient dans l'hémodialyseur.

A la sortie de l'hémodialyseur le liquide de dialyse traverse un élément de conduit transparent (30) coopérant avec un colorimètre d'un type connu en soi, logé dans la console (10).

Puis le liquide de dialyse pénètre par l'orifice (31) à la partie supérieure d'un bac (32) ouvert à l'atmosphère par un conduit (33) et dont le fond est relié de manière étanche par un conduit (34) au compartiment (24) de la poche placée dans le container (11). A l'intérieur du bac un flotteur (35), muni à son sommet d'un pointeau conique (36), coopère avec l'orifice (31) pour constituer un dispositif obturateur. Ce dispositif obturateur à flotteur régule automatiquement la pression du liquide de dialyse dans l'hémodialyseur. Un robinet (37) est raccordé au fond du bac (32). Un récipient gradué (38) est disposé sous le robinet (37) et le conduit (33). Le robinet (37) étant fermé, et le compartiment (24) étant d'abord vide d'air et n'offrant aucun volume disponible, le liquide pénètre dans le bac (32) jusqu'à ce que le flotteur obture l'orifice (31). Le circuit du liquide de dialyse est alors plein.

Avantageusement on procède simultanément au rinçage des compartiments de l'hémodialyseur destinés à être parcourus par le sang et par le liquide de dialyse. Le compartiment destiné au sang est rincé de façon conventionnelle, puis est raccordé au patient.

Pour rincer le compartiment destiné au liquide de dialyse, on tourne le robinet trois voies d'un nouveau tiers de tour pour le placer de la position 2 sur la position 3 et on met en route la pompe (28) qui déplace l'eau vers le compartiment complémentaire (24) et la remplace par le liquide de dialyse provenant du fond du compartiment (23) dans le container (11). Dès que, après quelques instants, le liquide de dialyse frais a rempli le compartiment correspondant de l'hémodialyseur et que, parallèlement, le patient est relié à l'hémodialyseur, le rein artificiel selon l'invention est prêt à fonctionner.

Le robinet trois voies reste sur la position 3 et la pompe (28) fait circuler à la vitesse désirée le liquide de dialyse frais. Le liquide de dialyse usagé occupe progressivement le compartiment (24) et déplace un égal volume de liquide frais dans le compartiment complémentaire (23) à l'intérieur du container (11).

Tout volume de liquide de dialyse recueilli dans le récipient gradué (38) par ouverture du robinet (37) est progressivement remplacé par un volume égal d'ultrafiltrat traversant la membrane de l'hémodialyseur, de sorte que le volume du liquide de dialyse dans le circuit à usage unique reste constant. On procède ainsi, à volonté, à l'hémodialyse et à l'ultrafiltration du sang. La séance est terminée lorsque la totalité du liquide de dialyse frais a été utilisée et déplacée dans le container (11) par un égal volume de liquide de dialyse usagé, grâce à la pompe (28) à travers l'hémodialyseur (12). Parallèlement on a prélevé dans le récipient gradué (38) une quantité de liquide de dialyse égale à la quantité d'ultrafiltrat désirée, selon le rythme et la fréquence désirés.

Généralement on procède à la vidange à l'égout du compartiment (24). Pour cela on peut par exemple tourner encore d'un tiers de tour le robinet (21) pour le replacer en position 1 et relier l'embout (17) à un dispositif capable de fournir de l'air sous une pression de l'ordre de quelques décimètres d'eau. Ce dispositif peut être constitué par une poche à clapet d'un type disponible dans le commerce, pouvant être actionnée à la main ou au pied. Puis on ouvre le robinet (37) dont on aura préalablement muni l'embout d'un conduit souple (non représenté) relié à l'égout. Sous l'effet de la pression d'air s'exerçant dans le compartiment (23), le liquide de dialyse usagé contenu dans le compartiment (24) remonte dans le bac (32) et s'écoule vers l'égout à travers la vanne (37), de préférence par siphonnage. On peut aussi amorcer simplement le siphon à l'aide du liquide de dialyse recueilli dans le récipient gradué (38).

Le matériel à usage unique peut alors être replacé dans le container (11) pour échange contre un ensemble identique neuf.

Les éléments à usage unique du rein artificiel selon l'invention sont essentiellement constitués de réservoirs rigides, de poches souples et d'éléments de tuyauterie de formes simples à fabriquer. Ils sont avantageusement réalisés en matériaux thermoplastiques bon marché mis en œuvre selon des techniques connues de moulage, d'injection, de découpage, permettant des fabrications de grande série à bas prix. En outre ils sont préalablement assemblés et contrôlés sur les lieux de fabrication, ce qui réduit très sensiblement les risques d'erreur à la mise en service.

Avantageusement la poche souple (14) est constituée par un film ou une gaine scellée sur les bords, en matériau pratiquement inextensible tel que le polyéthylène extrudé. Le container (11) est de préférence construit en matériau isolant thermiquement tel que le polystyrène ou le polyéthylène expansé, afin de conserver le mieux possible le liquide de dialyse à une température proche de la température d'utilisation.

Naturellement le rein artificiel selon la présente invention peut faire l'objet d'un grand nombre de variantes d'exécution à la portée du technicien.

Comme avantages du rein artificiel selon l'invention, on peut encore souligner l'économie de produits, de fluides ou de calories à mettre en œuvre à chaque séance. Ainsi par comparaison avec un rein artificiel conventionnel, le volume d'eau consommé est divisé par 5, donc le chauffage, la consommation d'adoucisseur et de concentré de dialyse sont également divisés par 5. De plus, on supprime toute consommation de liquides stérilisants.

En outre, ce qui est très apprécié des utilisateurs, notamment des patients à domicile, c'est la réduction des temps de préparation et de rangement du matériel à chaque séance.

Ainsi pour une séance d'hémodialyse d'une durée de 4 heures, avec un rein artificiel conventionnel, il faut ajouter 2 heures de préparation et de rangement, durée qui est considérablement réduite avec le rein selon l'invention. Ceci permet au patient qui, à domicile, commence son traitement trois fois par semaine à 18 heures, de le terminer bien avant minuit.

**Revendications**

1. Rein artificiel comprenant:

a) un hémodialyseur (12) divisé par une membrane permettant le traitement du sang par dialyse et par ultrafiltration en deux compartiments, le premier parcouru par le sang, le second par le liquide de dialyse,

b) des moyens pour faire circuler le sang traité dans ledit premier compartiment,

c) des moyens pour préparer le liquide de dialyse, le stocker, et le faire circuler dans ledit second compartiment,

d) des moyens pour prélever et mesurer des quantités de liquide égales aux quantités d'ultrafiltrat désirées,

e) des organes de commande et de contrôle desdits moyens selon b) et c), réutilisables et groupés sur une console (10) susceptible d'être reliée à une source de courant électrique, caractérisé en ce que lesdits moyens selon c) comprennent une cartouche à usage unique (20) de concentré de dialyse d'une capacité calibrée pour un traitement donné, apte à être raccordée à une source de distribution d'eau courante.

2. Rein artificiel selon la revendication 1, caractérisé en ce que lesdits moyens selon c) comprennent un adoucisseur d'eau (19) à usage unique susceptible d'être relié à une source de distribution d'eau courante.

3. Rein artificiel selon l'une quelconque des revendications précédentes, caractérisé en ce que lesdits moyens selon c) comprennent un robinet 3 voies (21) susceptible de relier deux à deux selon trois combinaisons différentes, un conduit d'introduction d'un mélange liquide capable de fournir du liquide de dialyse, une zone de stockage de liquide de dialyse et une pompe de circulation (28) dudit liquide de dialyse.

4. Rein artificiel selon l'une quelconque des revendications précédentes, dans lequel l'un au moins des moyens selon c) et/ou d) est préalablement interconnecté audit hémodialyseur (12) et forme avec celui-ci un ensemble stérile sous emballage étanche, prêt à l'emploi, caractérisé en ce que les moyens, nécessaires à une séance d'hémodialyse peuvent être mis en place et livré à l'intérieur d'un container (11) destiné à contenir pendant la durée du traitement, sans déformation sensible, le volume de liquide de dialyse frais et/ou usagé nécessaire audit traitement.

5. Rein artificiel selon la revendication 4, caractérisé en ce que lesdits moyens selon c) comprennent un tube plongeant (22) jusqu'au fond dudit container (11).

6. Rein artificiel selon la revendication 4, caractérisé en ce que ledit container (11) est destiné à servir de support aux différents moyens matériels constituant ledit rein artificiel, pendant la durée du traitement.

7. Rein artificiel selon la revendication 4, caractérisé en ce que ledit container (11) est construit en matériau thermiquement isolant.

8. Rein artificiel selon la revendication 4, caractérisé en ce que lesdits moyens selon c) comprennent une poche souple (14) à usage unique, préalablement vide d'air, susceptible d'occuper au cours du traitement la totalité du volume interne dudit container (11).

9. Rein artificiel selon la revendication 8, caractérisé en ce que ladite poche (14) est divisée de manière étanche en deux compartiments complémentaires (23) et (24), susceptibles d'occuper chacun ou ensemble, pendant la durée du traitement, la totalité du volume interne dudit container (11).

10. Rein artificiel selon la revendication 8, caractérisé en ce que ladite poche (14) est en matériau pratiquement inextensible.

11. Rein artificiel selon l'une quelconque des revendications précédentes, caractérisé en ce que lesdits moyens selon c) comprennent, en aval dudit second compartiment de l'hémodialyseur (12), un bac (32) ouvert à l'atmosphère, muni d'un dispositif relié à un flotteur (35) capable de réguler l'obturation du conduit le reliant à l'orifice de sortie du liquide de dialyse audit hémodialyseur et un conduit (34) reliant le fond dudit bac (32) audit container (11).

12. Rein artificiel selon la revendication 11, caractérisé en ce qu'il comporte en outre des moyens (37) pour prélever du liquide contenu dans ledit bac (32) et des moyens (38) pour mesurer le volume du liquide ainsi prélevé.

13. Rein artificiel selon l'une quelconque des revendications précédentes, caractérisé en ce que l'hémodialyseur (12), ainsi que lesdits moyens selon b), c), et/ou d) sont assemblés et maintenus fixés à l'aide de deux plaques (43, 44) parallèles, solidarisées mécaniquement.

14. Rein artificiel selon les revendications 6 et 13, caractérisé en ce que la première (43) desdites plaques peut être fixée sur ledit container (11), pendant la durée du traitement, délimitant ainsi le volume du liquide de dialyse.

15. Rein artificiel selon la revendication 13, caractérisé en ce que la seconde (44) desdites plaques peut recevoir ladite console (10) et la maintenir fixée, pendant la durée du traitement.

## Claims

1. An artificial kidney comprisjng:

a) a haemodialyser (12) divided by a membrane allowing the blood to be treated by dialysis and by ultrafiltration in two compartments, the first being traversed by the blood, the second by the dialysis liquid,

b) means for causing the blood treated in the first compartment to circulate,

c) means for preparing the dialysis liquid, storing it and causing it to circulate in the said second compartment,

d) means for drawing off and measuring quantities of liquid equal to the desired quantities of ultrafiltrate,

e) means for actuating and controlling the said means according to b) and c) which are reusable and grouped on a console (10) capable of being connected to an electric current source, characterised in that the said means in accordance with c) comprise a disposable cartridge (20) of the dialysis concentrate with a capacity calibrated for a given treatment, capable of being connected to a source dispensing running water.

2. An artificial kidney according to Claim 1, characterised in that the said means according to c) comprise a disposable water softener (19) capable of being connected to a source dispensing running water.

3. An artificial kidney according to any one of the preceding claims, characterised in that the said means according to c) comprise a three-way tap (21) capable of interconnecting in pairs according to three different combinations, a tube introducing a liquid mixture capable of supplying dialysis liquid, a storage zone for the dialysis liquid and a circulating pump (28) for the said dialysis liquid.

4. An artificial kidney according to any one of the preceding claims wherein at least one of the means according to c) and/or d) is first interconnected to the said haemodialyser (12) and forms, with the latter, a sterile unit in a leak proof pack ready for use, characterised in that the means necessary for a haemodialysis session can be positioned and delivered inside a container (11) intended to contain the volume of the fresh and/or used dialysis liquid required for the said treatment, during the period of the treatment, without any substantial deformation.

5. An artificial kidney according to Claim 4, characterised in that the said means according to c) comprise a tube (22) extending as far as the bottom of the said container (11).

6. An artificial kidney according to Claim 4, characterised in that the said container (11) is intended to serve as support for the various means of equipment constituting the said artificial kidney during the period of the treatment.

7. An artificial kidney according to Claim 4, characterised in that the said container (11) is made of a thermally insulating material.

8. An artificial kidney according to Claim 4, characterised in that the said means according to c) comprise a disposable flexible bag (14) previously emptied of air, capable of occupying in the course of the treatment the whole of the internal volume of the said container (11).

9. An artificial kidney according to Claim 8, characterised in that the said bag (14) is divided in a leak proof manner into two complementary compartments (23) and (24), capable of each occupying or jointly occupying, during the period of treatment, the whole of the internal volume of the said container (11).

10. An artificial kidney according to Claim 8, characterised in that the said bag (14) is made of a practically unstretchable material.

11. An artificial kidney according to any one of the preceding Claims, characterised in that the said means according to c) comprise, downline from the said second compartment of the haemodialyser (12) a tank (32) open to the atmosphere provided with a device connected to a float (35) capable of regulating the obturation of the tube connecting it to the outlet opening for the dialysis liquid on the said haemodialyser and a tube (34) connecting the bottom of the said tank (32) to the said container (11).

12. An artificial kidney according to Claim (11), characterised in that it comprises, moreover, means (37) for drawing off liquid contained in the said tank (32) and means (38) for measuring the volume of liquid thus drawn off.

13. An artificial kidney according to any one of the preceding claims, characterised in that the haemodialyser (12), as well as the said means according to b), c) and/or d) are assembled and kept fixed by means of two parallel plates (43, 44) which are mechanically interlocked.

14. An artificial kidney according to Claims 6 and 13, characterised in that the first (43) of the said plates can be fixed on the said container (11) during the period of the treatment, thus delimiting the volume of the dialysis liquid.

15. An artificial kidney according to Claim 13, characterised in that the second (44) of the said plates can accommodate the said console (10) and keep it fixed during the period of the treatment.

**Patentansprüche**

1. Künstliche Niere mit

a) einem Hämodialysator (12), der durch eine Membran unterteilt ist, die die Behandlung des Bluts durch Dialyse und durch Ultrafiltration in zwei Abteilen gestattet, von denen das erste vom Blut und das zweite von der Dialyseflüssigkeit durchströmt wird,

b) Mitteln zum Umwälzen des behandelten Bluts im ersten Abteil,

c) Mitteln zum Aufbereiten, Speichern und Umwälzen der Dialyseflüssigkeit im zweiten Abteil,

d) Mitteln zum Entnehmen und Messen von Flüssigkeitsmengen, die den gewünschten Ultrafiltratmengen gleich sind,

e) Steuer- und Kontrollorganen für die Mittel gemäss b) und c), die wiederverwendbar und auf einer Konsole (10) gruppiert sind, die mit einer elektrischen Stromquelle verbunden werden kann, dadurch gekennzeichnet, dass die Mittel gemäss c) eine für einmaligen Gebrauch bestimmte Kartusche (20) für Dialysekonzentrat und mit einem geeichten Fassungsvermögen für eine gegebene Behandlung aufweisen, die mit einer Verteilerquelle für fliessendes Wasser verbunden werden kann.

2. Künstliche Niere nach Anspruch 1, dadurch gekennzeichnet, dass die Mittel gemäss c) einen für einmaligen Gebrauch bestimmten Wasserenthärter (19) aufweisen, der mit einer Verteilerquelle für fliessendes Wasser verbunden werden kann.

3. Künstliche Niere nach einem der vorhergehenden Ansprüche, dadurch gekennzeichnet, dass die Mittel gemäss c) ein Dreiwegeventil (21) aufweisen, das paarweise und in drei unterschiedlichen Kombinationen verbinden kann: eine Einführungsleitung für ein flüssiges Gemisch, die Dialyseflüssigkeit liefern kann, eine Speicherzone für Dialyseflüssigkeit und eine Umwälzpumpe (28) für die Dialyseflüssigkeit.

4. Künstliche Niere nach einem der vorhergehenden Ansprüche, bei der wenigstens eines der Mittel gemäss c) und/oder d) zunächst mit dem Hämodialysator (12) verbunden ist und mit diesem eine sterile Anordnung in dichter verwendungsbereiter Verpackung bildet, dadurch gekennzeichnet, dass die für eine Hämodialysesitzung notwendigen Mittel in einen Behälter (11) eingebracht und geliefert werden können, der dazu bestimmt ist, während der Behandlungsdauer ohne merkliche Verformung das Volumen an für die Behandlung notwendiger frischer und/oder gebrauchter Dialyseflüssigkeit aufzunehmen.

5. Künstliche Niere nach Anspruch 4, dadurch gekennzeichnet, dass die Mittel gemäss c) ein Rohr (22) aufweisen, das bis zum Boden des Behälters (11) eintaucht.

6. Künstliche Niere nach Anspruch 4, dadurch gekennzeichnet, dass der Behälter (11) während der Behandlungsdauer dazu bestimmt ist, als Träger für verschiedene, die künstliche Niere bildende Geräte zu dienen.

7. Künstliche Niere nach Anspruch 4, dadurch gekennzeichnet, dass der Behälter (11) aus thermisch isolierendem Material hergestellt ist.

8. Künstliche Niere nach Anspruch 4, dadurch gekennzeichnet, dass die Mittel gemäss c) einen für den normalen Gebrauch bestimmten und zunächst luftleeren weichen Beutel (14) aufweisen, der im Verlauf der Behandlung das gesamte Innenvolumen des Behälters (11) einnehmen kann.

9. Künstliche Niere nach Anspruch 8, dadurch gekennzeichnet, dass der Beutel (14) dicht in zwei komplementäre Abteile (23 und 24) unterteilt ist, die jeweils oder gemeinsam während der Behandlungsdauer das gesamte Innenvolumen des Behälters (11) einnehmen können.

10. Künstliche Niere nach Anspruch 8, dadurch gekennzeichnet, dass der Beutel (14) aus einem praktisch nicht dehnbaren Material besteht.

11. Künstliche Niere nach einem der vorhergehenden Ansprüche, dadurch gekennzeichnet, dass die Mittel gemäss c) stromab des zweiten Abteils des Hämodialysators (12) ein zur Atmosphäre offenes Gefäss (32) aufweisen, das mit einer mit einem Schwimmer (35) verbundenen Vorrichtung versehen ist, die das Verschliessen der Leitung regeln kann, die das Gefäss mit der Auslassöffnung für Dialyseflüssigkeit am Hämodialysator verbindet, wobei eine Leitung (34) den Boden des Gefässes (32) mit dem Behälter (11) verbindet.

12. Künstliche Niere nach Anspruch 11, gekennzeichnet durch Mittel (37) zum Aufnehmen von im Gefäss (32) enthaltener Flüssigkeit und durch Mittel (38) zum Messen des Volumens der auf diese Weise aufgenommenen Flüssigkeit.

13. Künstliche Niere nach einem der vorhergehenden Ansprüche, dadurch gekennzeichnet, dass der Hämodialysator (12) und auch die Mittel gemäss b), c) und/oder d) durch zwei parallele mechanisch miteinander fest verbundene Platten (43, 44) zusammengebaut und festgehalten sind.

14. Künstliche Niere nach Anspruch 6 und 13, dadurch gekennzeichnet, dass die erste (43) der Platten während der Behandlungsdauer am Behälter (11) befestigt sein kann und auf diese Weise das Volumen der Dialyseflüssigkeit begrenzt.

15. Künstliche Niere nach Anspruch 13, dadurch gekennzeichnet, dass die zweite (44) der Platten während der Behandlungsdauer die Konsole (10) aufnehmen und festhalten kann.

Fig.1.

_Fig.2._

## Fig. 3.

## Fig. 4.

## Fig.5.

Fig.6.

0 049 673